# EUROPEAN PATENT SPECIFICATION

(11) **EP 0 847 989 B1**
(45) Date of publication and mention of the grant of the patent: **13.11.2002**
(21) Application number: 97400458.2
(22) Date of filing: 28.02.1997
(51) Int. Cl.: C07D 205/08, C07F 7/18

(54) **Process for producing cyclohexylazetidinone**
Verfahren zur Herstellung von Cyclohexylazetidinon
Procédé de préparation de cyclohexylazétidinone

(30) Priority: 12.12.1996 JP 33202196
(43) Date of publication of application: 17.06.1998
(73) Proprietor: Takasago International Corporation, Tokyo 108 (JP)
(72) Inventor: Matsumoto, Takaji, Takasago International Corp., Hiratsuka-shi, Kanagawa (JP); Murayama, Toshiyuki, Takasago International Corp., Hiratsuka-shi, Kanagawa (JP); Mitsuhashi, Shigeru, Takasago International Corp., Hiratsuka-shi, Kanagawa (JP); Miura, Takashi, Takasago International Corp., Hiratsuka-shi, Kanagawa (JP)
(74) Representative: Gillard, Marie-Louise

(56) References cited:
- EP-A- 0 416 952
- EP-A- 0 617 017
- WO-A-95/26333
- GB-A- 2 287 709
- G. KENNEDY ET AL.: "The use of zinc enolates in the synthesis of a key intermediate for the preparation of trinem antibiotics" TETRAHEDRON LETTERS, vol. 37, no. 41, 7 October 1996, OXFORD GB, pages 7441-7444, XP002057976
- C. GHIRON ET AL.: "The stereoselective synthesis of a key intermediate of the trinem antibiotic sanfetrinem" TETRAHEDRON LETTERS, vol. 37, no. 22, 27 May 1996, OXFORD GB, pages 3891-3894, XP002057977

## Description

The present invention relates to a process for producing a cyclohexylazetidinone derivative which is useful as an intermediate for synthesis of tricyclic β-lactam antibiotics (trinem antibiotics).

Tricyclic β-lactam compounds (trinem) are known to have excellent antibacterial activities (EP 0416953 A2). A typical example of such trinem compounds is Sanfetrinem having the following formula (VI):

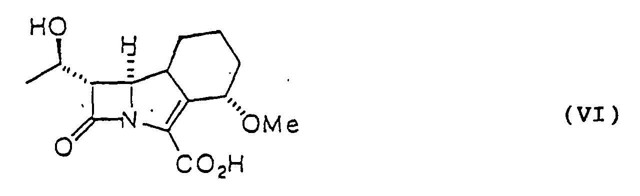

For the synthesis of this compound, a compound having the following formula (IV): 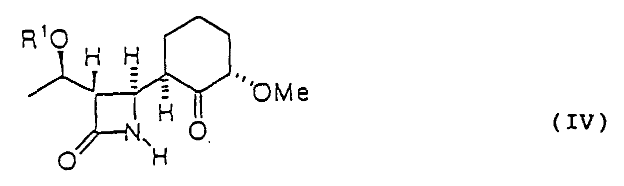 in which R¹ is a trialkylsilyl group;
is a key intermediate G. Kennedy et al, Tetrahedron Letters, 37, 7441 (1996) and C. Ghiron et al Tetrahedron Letters, 37, 3891 (1996).

In order to synthesise such intermediate product, the following methods have typically been disclosed.

A. Perboni et al. (EP 0466509 A1) disclosed a process in which acetoxyazetidinone (IIIa) is coupled with 1-trimethylsilyloxycyclohexene in the presence of a Lewis acid catalyst to prepare cyclohexanone, and subsequently, reduction to cyclohexene, stereoselective epoxidation, methanolysis, and oxidation are performed, as shown in the following scheme:

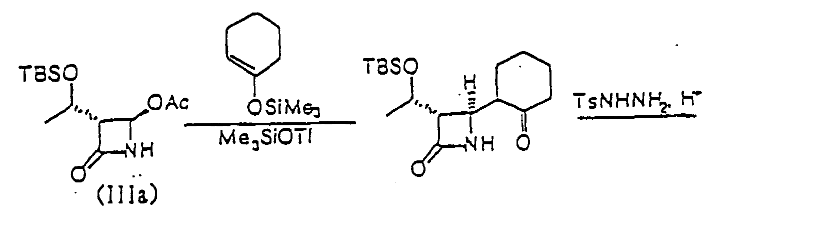

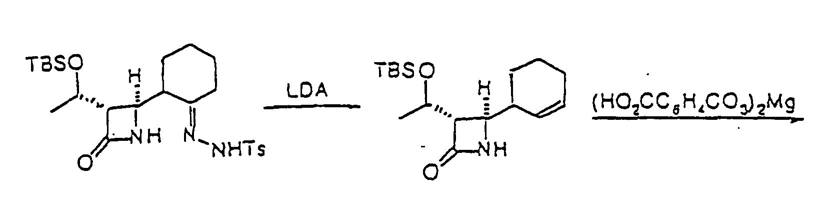

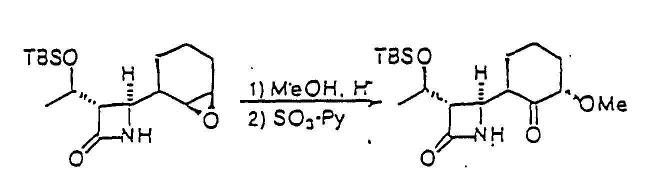

As to introducing cyclohexene into the structure of acetoxyazetidinone (IIIa) in the above process, S. Biondi et al. (EP 617017 A1) and T. Rossi et al. [*J. Am. Chem. Soc*., 117, 9604 (1995)] reported methods using allylborane in the presence of Lewis acid, as shown in the following scheme:

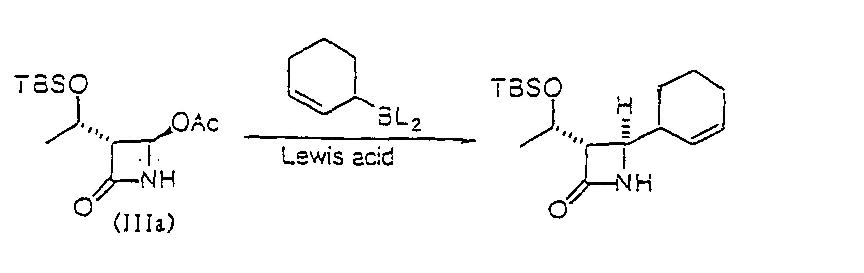

Further, T. Rossi et al. (WO 95/26333) disclosed a method for direct condensation of azetidinone (IIIa) with (2S)-2-methoxycyclohexanone using stannic chloride, as- shown in the following scheme:

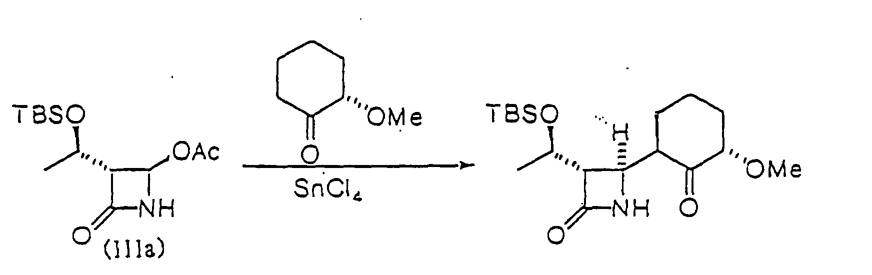

Further, A. Pecunioso et al. (GB 2287709 A) disclosed a method using stannic chloride for coupling with silyl enol ether, as shown in the following scheme:

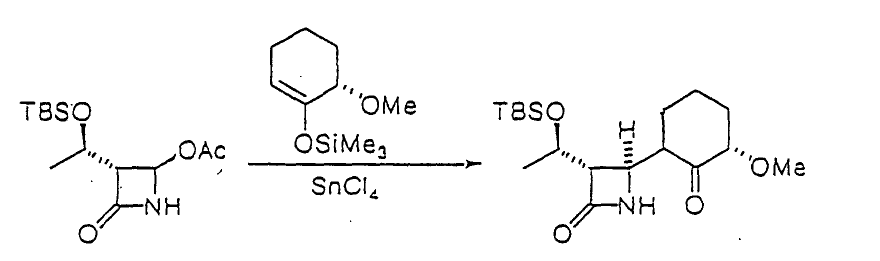

However, the above-mentioned methods do have some drawbacks. For example, the method of A. Perboni et al. has a relatively large number of reaction steps, so the yield is therefore low. In the methods using Lewis acid as proposed by S. Biondi et al., T. Rossi et al., and A. Pecunioso et al. a large amount of Lewis acid is used, which requires a great deal of work in post-treatment, especially in'the disposal of waste fluid.

Therefore, research has been carried out into industrial processes for synthesising cyclohexylazetidinone (IV). As a result, a novel and advantageous process has been found as described below.

Accordingly, the object of the present invention is to provide a process for producing cyclohexylazetidinone (IV). Specifically, the process of the present invention comprises treating (2S)-2-methoxycyclohexanone having the formula (I) with a base to prepare a magnesium enolate derivative having the formula (II), and condensing the magnesium enolate derivative with acyloxyazetidinone having the formula (III) to stereoselectively produce the sought-after cyclohexylazetidinone (IV), as shown in the following scheme: 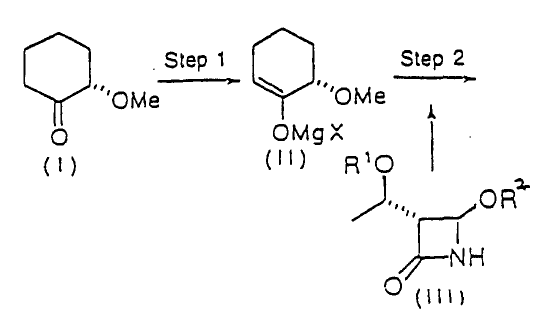 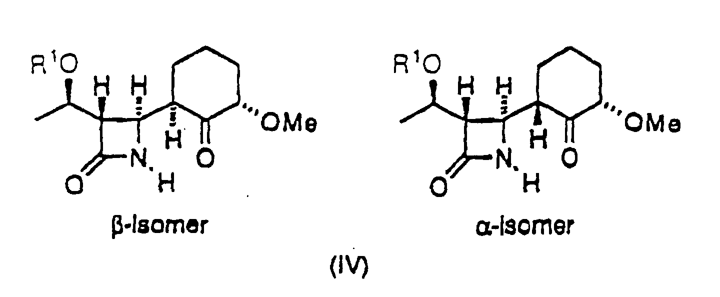 wherein R¹ is a hydroxyl-protecting group; R² is an acyl group; and X is a halogen atom.

To this end, the invention provides a process for.producing a cyclohexylazetidinone derivative having the general formula (IV): 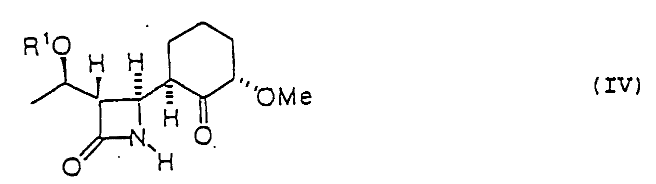 in which R¹ is a hydroxyl-protecting group and Me is a methyl group.

The process comprises the step of
reacting (3R,4R)-4-acyloxy-3-[(R)-1-hydroxyethyl]-azetidin-2-one derivative having the general formula (III): 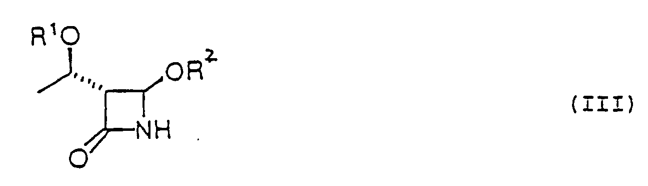 in which R¹ is the same group as described above and R² is an acyl group;
with a magnesium enolate compound having the general formula (II): 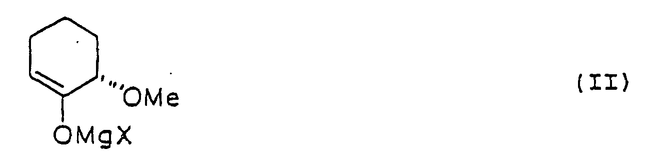 in which X is a halogen atom;
in a solvent.

Preferably, the solvent is either butyl acetate or toluene.

The magnesium enolate compound may be obtained by reacting (2S)-2-methoxycyclohexanone having the general formula (I): 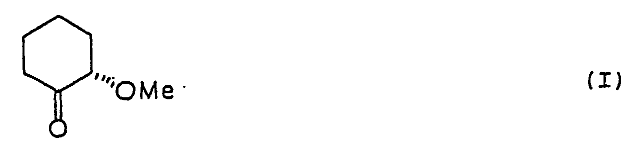 where Me indicates a methyl group;
either with a product obtained by reacting a Grignard reagent with an organic base, or with a Grignard reagent in the presence of an organic base.

The product obtained by reacting a Grignard reagent with an organic base may be a magnesium amide having the general formula (V):

R³R⁴NMgX (V)

where each of R³ and R⁴ is a lower alkyl group having 1 to 6 carbon atoms, a lower alkenyl group having 1 to 6 carbon atoms, a substituted or non-substituted aralkyl group, or a substituted or non-substituted phenyl group, R³ and R⁴ being identical or different;
and X is a halogen atom.

The organic base may be a secondary amine having the general formula (VII)

R³R⁴NH (VII)

where R³ and R⁴ have the same meaning as described above, and;
said Grignard reagent has the general formula (VIII):

R⁵MgX (VIII)

where R⁵ has the same meaning as that of R³ or R⁴, the groups R³, R⁴ and R⁵ being identical or different; and X is a halogen atom.

Preferably, the organic base or secondary amine is either diethylamine or diisopropylamine.

In another embodiment, the magnesium enolate compound may be obtained by reacting a corresponding 2-halogeno cyclohexanone with metallic magnesium.

In a further another embodiment, the magnesium enolate compound may be obtained by reacting a corresponding lithium enolate derivative with a magnesium halide.

According to still another embodiment, the magnesium enolate compound may be obtained by reacting a corresponding o-trialkylsilyl enol ether derivative with an alkyl magnesium halide.

The base used in the present invention is a magnesium amide having the general formula (V):

R³R⁴NMgX (V)

which is prepared by reacting a secondary amine having the general formula (VII):

R³R⁴NH (VII)

wherein each of R³ and R⁴ is a lower alkyl group having 1 to 6 carbon atoms, a lower alkenyl group having 1 to 6 carbon atoms, a substituted or non-substituted aralkyl group, or a substituted or non-substituted phenyl group, and R³ and R⁴ may be identical or different;
with a Grignard reagent having the general formula (VIII):

R⁵MgX (VIII)

wherein R⁵ is a lower alkyl group having 1 to 6 carbon atoms, a lower alkenyl group having 1 to 6 carbon atoms, a substituted or non-substituted aralkyl group, or a substituted or non-substituted phenyl group, and X is a halogen atom.

According to the present invention, (2S)-2-methoxycyclohexanone (I) may first be transformed into a magnesium enolate derivative (II) of (2S)-2-methoxycyclohexanone. The condensation reaction between the magnesium enolate derivative (II) and acyloxyazetidinone (III) is then carried out to obtain tricyclic β-lactam intermediates (IV). The process of the present invention does not involve any use of Lewis acid, so that no impedient waste fluid is formed. The sought-after cyclohexylazetidinone derivatives can thus be produced in an industrially advantageous and simple manner.

The above and other objects, features and advantages of the invention will be made apparent from the following description of the preferred embodiments, given as a non-limiting example.

The hydroxyl-protecting group used as R¹ in the formulae (III) and (IV) may include, for example, *tert*butyldimethylsilyl group, trimethylsilyl group, or the like.

The acyl group used as R² in the formula (III) may comprise, for example, an alkanoyl group having 1 to 9 carbon atoms, a cycloalkanoyl group having 4 to 7 carbon atoms, a benzoyl group, a 5-membered or 6-membered heteroaroyl group, etc..

The alkanoyl group used as substituent R² in the formula (III) includes formyl group, acetyl group, propionyl group, butanoyl group, pentanoyl group, hexanoyl group, heptanoyl group or octanoyl group.

The cycloalkanoyl group includes cyclopropylcarbonyl group, cyclopentylcarbonyl group or cyclohexylcarbonyl group.

The heteroaroyl group includes heteroaroyl group having one or more nitrogen atoms, oxygen atoms or sulfur atoms. Preferred heteroaroyl group includes furoyl group, thiophenecarbonyl group or nicotinyl group.

The secondary amine to be used in the above-mentioned reaction may include, for example: diethylamine, diisopropylamine, dipropylamine, dibutylamine, diisobutylamine, di-tertiary-butylamine, dicyclohexylamine, diphenylamine, dibenzylamine, dicyclopentylamine, dihexylamine, methylethylamine, ethylpropylamine, ethylisopropylamine, morpholine, piperidine, pyrrolidine, indole, and 1,1,1,3,3,3-hexamethyldisilazane. Among these compounds, diethylamine and diisopropylamine are preferably used. However, diisopropylamine is most preferably used. The amount of secondary amine to be used ranges from 1 to 2 molar equivalents, preferably 1.1 to 1.3 molar equivalents, relative to one mole of the Grignard reagent.

The Grignard reagent may comprise, for example: methylmagnesium chloride, methylmagnesium bromide, methylmagnesium iodide, ethylmagnesium chloride, ethylmagnesium bromide, ethylmagnesium iodide, propylmagnesium chloride, propylmagnesium bromide, propylmagnesium iodide, isopropylmagnesium chloride, isopropylmagnesium bromide, isopropylmagnesium iodide, butylmagnesium chloride, butylmagnesium bromide, butylmagnesium iodide, isobutylmagnesium chloride, isobutylmagnesium bromide, isobutylmagnesium iodide,. tert-butylmagnesium chloride, *tert*-butylmagnesium bromide, tert-butylmagnesium iodide, phenylmagnesium chloride, phenylmagnesium bromide, phenylmagnesium iodide, benzylmagnesium chloride, benzylmagnesium bromide, benzylmagnesium iodide, vinylmagnesium chloride, allylmagnesium chloride, and allylmagnesium bromide. Among these Grignard reagents, ethylmagnesium bromide, isopropylmagnesium bromide, and tert-butylmagnesium chloride are preferably used. However, tert-butylmagnesium chloride is most preferably used. The amount of the Grignard reagent to be used ranges from 1 to 1.5 molar equivalents, preferably 1.05 to 1.2 molar equivalents, relative to one mole of methoxycyclohexanone.

A practical way to prepare magnesium amide is to add the Grignard reagent to the secondary amine, or vice versa. The reaction temperature for the preparation may vary from -30°C to +100°C, but is preferably in the range of 0°C to 20°C. The reaction time may vary from a few minutes to 24 hours, but is preferably in the range of 30 minutes to 1 hour. Any solvent which does not affect the reaction can be used as the solvent for the preparation of magnesium amide. Further, each solvent may be used sole or as a mixture with other solvents. Such a solvent may be selected from the group consisting of: hydrocarbons such as hexane, heptane, octane, nonane, decane, benzene, toluene, and xylene; halogenated hydrocarbons such as dichloromethane, chloroform, and 1,2-dichloroethane; ethers such as tetrahydrofuran, dioxane, dimethoxyethane, diisopropyl ether, diethylene glycol dimethyl ether; and acetonitrile. Preferably, the solvent is selected from the group consisting of ethers such as tetrahydrofuran, dioxane, dimethoxyethane, diisopropyl ether and diethylene glycol dimethyl ether. Among them, tetrahydrofuran is the most preferred solvent.

A magnesium enolate derivative is then prepared by adding (2S)-2-methoxycyclohexanone to the above-prepared magnesium amide. The reaction temperature for the preparation may range from -30°C to +50°C, but is preferably in the range of 0°C to 10°C. The reaction time may range from a few minutes to 24 hours, but is preferably in the range of 10 minutes to 3 hours. Any solvent which does not affect the reaction can be used as the solvent for the preparation of magnesium enolate derivative. Each solvent may be used sole or as a mixture with other solvents. Such a solvent may be selected from the group consisting of hydrocarbons such as hexane, heptane, octane, nonane, decane, benzene, toluene, and xylene; halogenated hydrocarbons such as dichloromethane, chloroform, and 1,2-dichloroethane; ethers such as tetrahydrofuran, dioxane, dimethoxyethane, diisopropyl ether, diethylene glycol dimethyl ether; and acetonitrile. Preferably, the solvent is selected from the group consisting of ethers such as tetrahydrofuran, dioxane, dimethoxyethane, diisopropyl ether and diethylene glycol dimethyl ether. Among them, tetrahydrofuran is the most preferred solvent. Though not being especially limited, the amount of solvent to be used usually ranges from 2-fold to 50-fold by weight, preferably from 5-fold to 10-fold by weight, of (2S)-2-methoxycyclohexanone. Formation of the magnesium enolate derivative in this process can be confirmed by effecting silyl enol etherification of the product with trimethylchlorosilane, isolating the silylenolether produced and analysing it by ¹H-NMR.

Alternatively, the magnesium enolate compound (II) can also be prepared, for example, by the following method (1), (2), or (3):
(1) Treating a 2-halocarbonyl compound with metal magnesium, as shown in the scheme below (P. Fellmann et al., Tetrahedron, 1978, 1349); 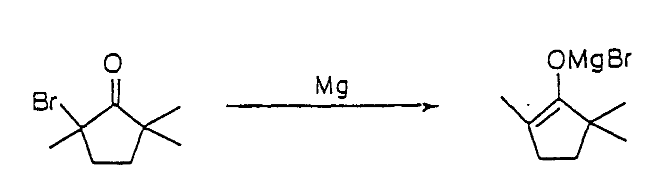
(2) Preparing a lithium enolate compound from lithium diisopropylamide or the like, and exchanging the metal with a magnesium halide, as shown in the scheme below (G. Stork et al., *J. Am. Chem. Soc.*, 1968, 4464); 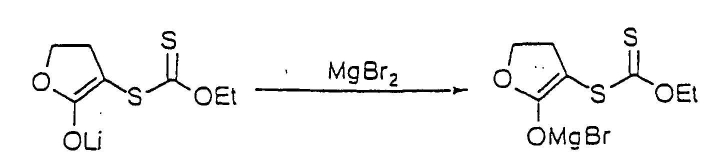 and
(3) Reacting silyl enol ether with a Grignard reagent (S. Matsui, et al., *Bull. Chem. Soc. Jpn.,* 1987, 1853), as shown in the scheme below.

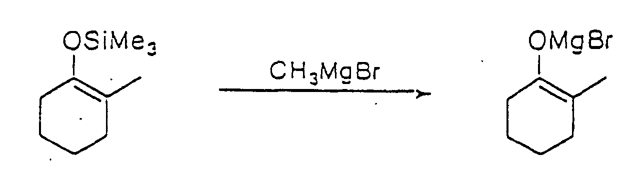

According to method (2), for example, (2S)-2-methoxycyclohexanone is reacted with lithium diisopropylamide to obtain a lithium enolate derivative, and the lithium enolate derivative is reacted with magnesium bromide in order to prepare the sought-after magnesium enolate derivative (II). According to the method (3), the sought-after magnesium enolate derivative (II) can be prepared, for example, by reacting 1-trimethylsilyoxy-6-(S)-methoxy-1-cyclohexene, which can be synthesised from (2S)-2-methoxycyclohexanone, with methylmagnesium bromide.

As described above, the method and conditions for preparing the magnesium enolate derivative are not especially limited, and they may be selected from the view point of labour saving, cost saving, or the like.

According to the invention, the magnesium enolate derivative (II) may first be prepared, then condensed with acyloxyazetidinone (III). When preparing the magnesium enolate derivative (II) in step 2, secondary amines may be formed. These amines may preferably be removed from the reaction mixture of the magnesium enolate derivative (II) prior to the condensation reaction, so that these amines do not affect the condensation reaction and lower the reaction yield. The amount of magnesium enolate derivative (II) to be used in the reaction may vary from 1.0 to 1.5 molar equivalents, preferably from 1.05 to 1.2 molar equivalents, relative to one mole of acyloxyazetidinone (III). The solvent to be used in the condensation may be selected from the group consisting of: hydrocarbons such as hexane, heptane, octane, nonane, decane, benzene, toluene, and xylene; halogenated hydrocarbons such as dichloromethane, chloroform, and 1,2-dichloroethane; ethers such as tetrahydrofuran, dioxane, dimethoxyethane, diisopropyl ether, diethylene glycol dimethyl ether; esters such as ethyl acetate, propyl acetate, isopropyl acetate, and butyl acetate; N,N-dimethylformamide; dimethylsulfoxide; and acetonitrile. Preferably, the solvent is selected from the group consisting of: hydrocarbons such as benzene, toluene, xylene; ethers such as tetrahydrofuran, dioxane, dimethoxyethane, diisopropyl ether, diethylene glycol dimethyl ether; or esters such as ethyl acetate, propyl acetate, isopropyl acetate, and butyl acetate. Still more preferable, the solvent is butyl acetate or toluene. The reaction is normally performed at a temperature ranging from -30°C to +40°C, but is preferably in the range of -10°C to +20°C. Though not being especially limited, the amount of solvent to be used is normally 2-fold to 50-fold by weight, preferably 5-fold to 10-fold by weight of (2S)-2-methoxycyclohexanone.

According to the present invention, a magnesium enolate derivative (II) of (2S)-2-methoxycyclohexanone is first prepared from (2S)-2-methoxycyclohexanone (I). The magnesium enolate derivative (II) is then condensed with acyloxyazetidinone (III) to obtain an intermediate (IV) of a tricyclic β-lactam compound. The process of the present invention does not require any use of Lewis acid, so that no cumbersome waste fluid is formed. The sought-after cyclohexylazetidinone derivatives can therefore be produced in an industrially advantageous and simple manner.

The present invention will be illustrated in detail with the examples below.

Apparatuses and materials used for measurements are as follows:
- Melting point measuring apparatus: MP-500D manufactured by Yanako Institute for Apparatus Development;
- ¹H NMR spectrum measuring apparatus: AM-400 (400 MHz) manufactured by Bruker Corporation, and Gemini-2000 (200 MHz) manufactured by Varian Corporation;
- Internal Standard Substance: tetramethylsilane (in CDCl₃).

Gas chromatography for detecting the disappearance of (1S,2S)-2-methoxycyclohexanol was carried out under the following conditions:
- Apparatus: HP-5890 series II Plus manufactured by Hewlett Packard Co.;
- Column: Neutrabond-1 (0.25 mm x 30 m) manufactured by GL Sciences Co., Ltd.;
- Carrier gas: Helium
   * Injection temperature: 220°C
   * Detection temperature: 250°C
   * Oven temperature: initial 80°C through final 250°C with a heating rate of 2°C/min.

Gas chromatography for measuring the optical purity of (2S)-2-methoxycyclohexanone was carried out under the following conditions:
- Apparatus: GC-14A manufactured by Shimadzu Corporation;
- Column: Chiraldex B-TA (0.25 mm x 30 m) manufactured by Astec Corporation;
- Carrier gas: Helium
   * Injection temperature: 200°C
   * Detection temperature: 250°C
   * Oven temperature: 90°C

High performance liquid chromatography for measuring diastereoselectivity was carried out under the following conditions:
- Detector: L-4000 UV Detector manufactured by Hitachi Ltd.;
- Pump: L-6000 Pump manufactured by Hitachi Ltd.;
- Column: Inertsil ODS-2 (4.6 mm x 250 mm) manufactured by GL Sciences Co., Ltd.;
- Eluting solution: acetonitrile/water = 70/30
- Flow rate: 1.0 ml/min.;
- Detecting wavelength: 205 nm;
- Internal standard: n-Hexyl benzoate manufactured by Tokyo Kasei Kogyo Co., Ltd.

### EXAMPLE 1 (Prior art)

### Synthesis of (2S)-2-Methoxycyclohexanone (I)

The starting material (2S)-2-methoxycyclo hexanone was synthesised from (1S,2S)-2-methoxycyclohexanol by a known method as disclosed in GB 2287709A or WO 95/26333.

To a 1 litre 4-neck flask, (1S,2S)-2-methoxycyclohexanol (53.57 g, 0.411 mol) and methylene chloride (250 ml) were placed. Subsequently, 2,2,6,6-tetramethyl-1-piperidinyloxy free radical (1.28 g, 0.008 mol), potassium bromide (1.47 g, 0.0123 mol), and 8% sodium hydrogencarbonate solution (52 ml, 0.0494 mol) were added and cooled to 0°C. To this mixture, 3.1 N sodium hypochlorite solution (254 ml, 0.787 mol) was added dropwise over a period of 6 hours while maintaining the temperature between O°C and 5°C. After the disappearance of raw material (1S,2S)-2-methoxycyclohexanol, confirmed by gas chromatography, sodium sulfite (5.36 g, 0.0425 mol) was added to the mixture prior to separation. Distillation was then performed, and the aqueous phase was subjected to extraction with two 25 ml portions of methylene chloride. The solvent was distilled off under a reduced pressure, and further distillation was performed to obtain (2S)-2-methoxycyclohexanone (51.67 g).

The yield, boiling point and optical purity of the product were respectively 95%, 84°C/24 mmHg and 100% by enantiometric excess ratio.

### EXAMPLE 2 (Prior art)

### Synthesis of 1-trimethylsilyloxy-6-(S)-methoxy-1-cyclohexene from (2S)-2-methoxycyclohexanone (I).

A tetrahydrofuran solution (12.3 ml, 21 mmol) containing 1.7 mol/litre of tert-butylmagnesium chloride was added dropwise to a solution of diisopropylamine (3.08 ml, 22 mmol) in tetrahydrofuran (10 ml) which had been cooled to 0°C, and the mixture was stirred for 30 min. To this mixture, a solution of (2S)-2-methoxycyclohexanone (2.56 g, 20 mmol) in tetrahydrofuran (50 ml) was added dropwise at 0°C over a period of 30 min., and the resulting mixture was stirred at 0°C for a further 30 min. To the mixture, trimethylchlorosilane (2.79 ml, 22 mmol) was added dropwise. The mixture was stirred at 0°C for 30 min., and further stirred at room temperature for 2 hours. The reaction mixture was then added to an ice-cold ammonium chloride solution (50 ml), and subjected to extraction with hexane (50 ml). The resultant organic phase was washed with two 50 ml portions of water and dried with anhydrous magnesium sulfate. The solvent was then distilled off under atmospheric pressure. The remaining solution was then distilled under a reduced pressure to obtain a colourless, transparent and oily product of l-trimethylsilyloxy-6-(S)-methoxy-1-cyclohexene.

The yield and boiling point of the product were respectively 75% and 86°C/15 mmHg.

### EXAMPLE 1

### Synthesis of (3S,4R)-3-[(R)-1-((tert-butyldimethylsilyl)oxy)ethyl]-4[(1R,3S)-3-methoxy-2-oxocyclohexyl]azetidin-2-one

To a solution of diisopropylamine (1.96 ml, 14 mmol) in tetrahydrofuran (10 ml) which had been cooled to 0°C, a tetrahydrofuran solution (6.49 ml, 12 mmol) containing 1.85 mol/litre of *tert*-butylmagnesium chloride was added dropwise and stirred for 30 min. To this mixture, a solution of (2S)-2-methoxycyclohexanone (1.41 g, 11 mmol) in tetrahydrofuran (10 ml) was added dropwise at 0°C over a period of 20 min. After the reaction mixture was stirred at 0°C for 10 min., the solvent and the amine produced in the reaction mixture were completely distilled off. To the residue, butyl acetate (15 ml) was added and cooled to 5°C. Afterwards, a solution of (3R,4R)-4-acetoxy-3-[(R)-1-tert-butyldimethylsilyloxyethyl]azetidin-2-one (2.87 g, 10 mmol) in butyl acetate (15 ml) was added dropwise at 5°C over a period of 20 min., and stirred for a further 30 min. Methanol (10 ml) was then added and stirred for 10 min. Subsequently, 2 N hydrochloric acid was added for separation. The organic phase was washed with a saturated sodium hydrogencarbonate solution and water, and concentrated under a reduced pressure to obtain a crude product of (3S,4R)-3-[(R)-1-((*tert*-butyldimethylsilyl)oxy)ethyl]-4[(1R,3S)-3-methoxy-2-oxocyclohexyl]azetidin-2-one.

The crude product contained 3.02 g of the sought-after β-isomer (IV). The yield was 85%, and the β:α ratio was 92:8.

The diastereoselectivity of the synthesised cyclohexylazetidinone derivative was examined by high performance liquid chromatography. The yield and purity of the synthesised cyclohexylazetidinone derivative were determined on the basis of an internal standard.

The above-obtained crude product of the cyclohexylazetidinone derivative was recrystallised by dissolving in heptane to obtain 2.31 g of white crystal compound. The physicochemical properties of the crystal compound were found to be similar to those reported in WO 95/26333 by T. Rossi et al., and are as follows:
- Melting point: 132°C to 133°C;
- ¹H NMR (400 MHz, CDCl₃) δ: 5.86(s, 1H), 4.19(m, 1H), 3.99(m, 1H), 3.57(t, 1H), 3.28(s, 3H), 3.09(m, 1H), 2.88(m, 1H), 2.23(m, 1H), 2.10(m, 1H), 1.99(m, 1H), 1.74-1.6(m, 2H), 1.58(m, 1H), 1.25(d, 3H), 0.87(s, 9H), 0.08(s, 3H), 0.06(s, 3H).

### EXAMPLE 2

(3S,4R)-3-[(R)-1-((*tert*-butyldimethylsilyl)oxy)ethyl]-4[(1R,3S)-3-methoxy-2-oxocyclohexyl]azetidin-2-one was synthesised as a crude product by the same procedure as in Example 1, except that tetrahydrofuran was used as the solvent, instead of butyl acetate, for the condensation reaction with (3S,4R)-4-acetoxy-3-[(R)-1-*tert*-butyldimethylsilyloxyethyl]azetidin-2-one.

The crude product contained 2.63 g of the sought-after β-isomer (IV). The yield was 74%, and the β:α ratio was 87:13.

### EXAMPLE 3

(3S,4R)-3-[(R)-1-((*tert*-butyldimethylsilyl)oxy)ethyl]-4[(1R,3S)-3-methoxy-2-oxocyclohexyl]azetidin-2-one was synthesised as a crude product by the same procedure as in Example 1, except that diethylamine (1.45 ml, 14 mmol) was used instead of diisopropylamine, and that tetrahydrofuran was used as the solvent, instead of butyl acetate, for the condensation reaction with (3S,4R)-4-acetoxy-3-[(R)-1-*tert*-butyldimethylsilyoxyehtyl]azetidin-2-one.

The crude product contained 2.70 g of the sought-after β-isomer (IV). The yield was 76%, and the β:α ratio was 86:14.

### EXAMPLES 4 TO 9

In similar procedures to those used in Examples 1 to 3 above, the sought-after cyclohexylazetidinone was produced using various secondary amines and solvents, and under various reaction conditions, as shown in Table 1. The yields and the β:α ratios of the obtained products are also shown in Table 1.

### EXAMPLE 10

To a solution of diisopropylamine (1.96 ml, 14 mmol) in tetrahydrofuran (10 ml) which had been cooled to 0°C, a 1.6 M butyllithium solution (7.5 ml) was added dropwise and stirred for 30 min. To this mixture, a solution of (2S)-2-methoxycyclohexanone (1.41 g, 11 mmol) in tetrahydrofuran (10 ml) was added dropwise at 0°C and stirred for 30 min. Subsequently, magnesium bromide (2.21 g, 12 mmol) was added and stirred for a further 30 min. The reaction mixture was concentrated under a reduced pressure to give a residue. The residue was dissolved in tetrahydrofuran (15 ml) and cooled to 0°C. A solution of (3R,4R)-4-acetoxy-3-[(R)-1-*tert*-butyldimethylsilyloxyethyl]azetidin-2-one (2.87 g, 10 mmol) in tetrahydrofuran (15 ml) was then added dropwise and stirred for 1 hour. To the resultant mixture, methanol (10 ml) and 2 N hydrochloric acid (10 ml) were added for separation. The organic phase was washed with a saturated sodium hydrogencarbonate solution (10 ml), then with water (10 ml), and concentrated under a reduced pressure to obtain a crude product of (3S,4R)-3-[(R)-1-((tert-butyldimethylsilyl)oxy)ethyl]-4[(1R,3S)-3-methoxy-2-oxocyclohexyl]azetidin-2-one.

The crude product contained 710 mg of the sought-after β-isomer (IV). The yield was 20%, and the β:α ratio was 72:28.

### EXAMPLE 11

### Synthesis of (3S,4R)-3-[(R)-1-((tert-butyldimethylsilyl)oxy)ethyl]-4[(1R,3S)-3-methoxy-2-oxocyclohexyl]azetidin-2-one using 1-trimethylsilyloxy-6-(S)-methoxy-1-cyclohexene.

To a solution of 1-trimethylsilyloxy-6-(S)-methoxy-1-cyclohexene (800 mg, 4 mmol) in tetrahydrofuran (5 ml), a tetrahydrofuran solution containing 1 mol/litre of ethylmagnesium bromide was added and stirred at room temperature for 3 days. This mixture was then cooled to 0°C, and a solution of (3R,4R)-4-acetoxy-3-[(R)-1-*tert*-butyldimethylsilyloxyethyl]azetidin-2-one (861 mg, 3 mmol) in tetrahydrofuran (5 ml) was added dropwise to the mixture and stirred at 0°C for 1 hour. To the resultant mixture was added 2 N hydrochloric acid and the mixture was extracted with butyl acetate. The organic phase was washed with a saturated sodium hydrogencarbonate solution, then with water, and concentrated under a reduced pressure to obtain a crude product of (3S,4R)-3-[(R)-1-((tert-butyldimethylsilyl)oxy)ethyl]-4[(1R,3S)-3-methoxy-2-oxocyclohexyl]azetidin-2-one.

The crude product contained 170 mg of the sought-after β-isomer. The yield was 16%, and the β:α ratio was 90:10.

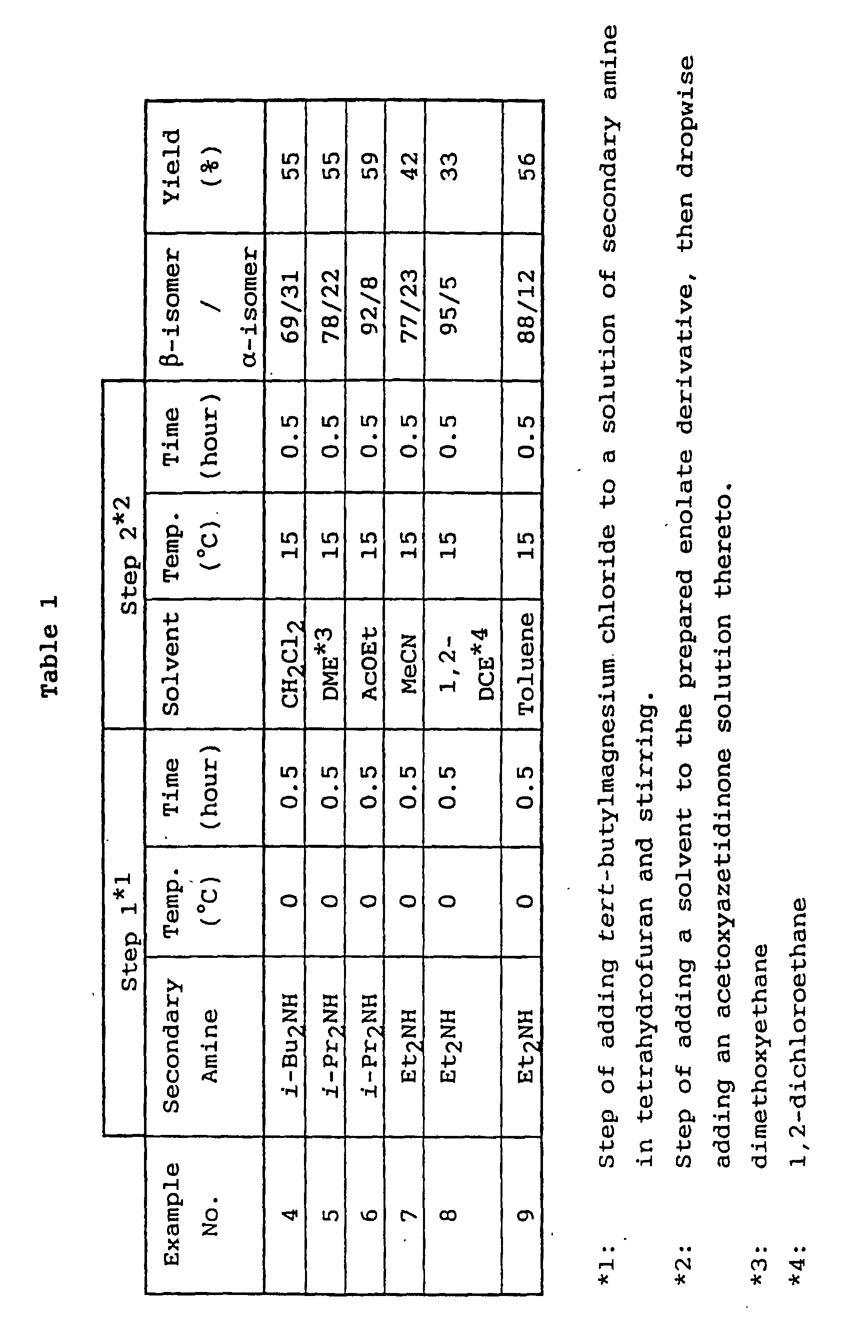

## Claims

1. A process for producing a cyclohexylazetidinone derivative having the general formula (IV): 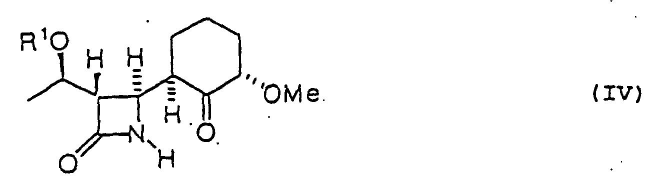 in which R¹ is a hydroxyl-protecting group and Me is a methyl group,
**characterised in that** said process comprises the step of
reacting (3R,4R)-4-acyloxy-3-[(R)-1-hydroxyethyl]-azetidin-2-one derivative having the general formula (III): 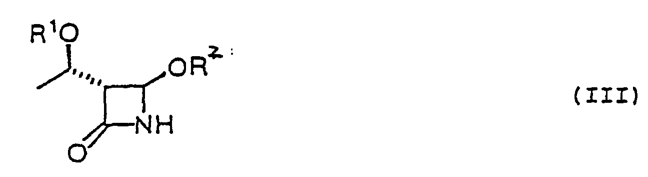 in which R¹ is the same group as described above and R² is an acyl group;
with a magnesium enolate compound having the general formula (II): 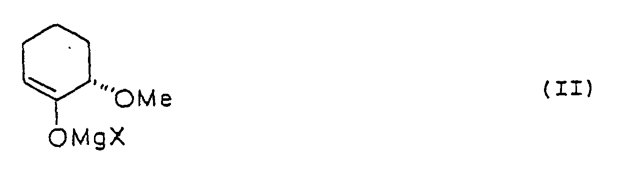 in which X is a halogen atom and Me is a methyl group; in a solvent.

2. The process according to claim 1, wherein said solvent is either butyl acetate or toluene.

3. The process according to claim 1 or 2, wherein said magnesium enolate compound is obtained by reacting (2S)-2-methoxycyclohexanone having the general formula (I): 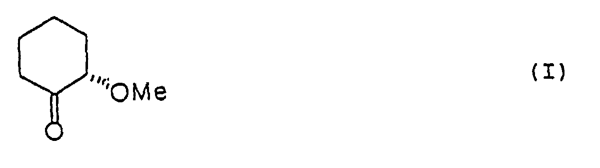 where Me is a methyl group; either with a product obtained by reacting a Grignard reagent with an organic base, or with a Grignard reagent in the presence of an organic base.

4. The process according to claim 3, wherein said product obtained by reacting a Grignard reagent with an organic base is a magnesium amide having the general formula (V):
R³R⁴NMgX (V)
where each of R³ and R⁴ is a lower alkyl group having 1 to 6 carbon atoms, a lower alkenyl group having 1 to 6 carbon atoms, a substituted or non-substituted aralkyl group, or a substituted or non-substituted phenyl group, R³ and R⁴ being identical or different;
and X is a halogen atom.

5. The process according to claim 3, wherein said organic base is a secondary amine having the general formula (VII)
R³R⁴NH (VII)
where R³ and R⁴ have the same meaning as in claim 4, and;
said Grignard reagent has the general formula (VIII):
R⁵MgX (VIII)
where R⁵ has the same meaning as that of R³ or R⁴, the groups R³, R⁴ and R⁵ being identical or different; and
X is a halogen atom.

6. The process according to any one of claims 3 to 5, wherein said organic base or secondary amine is either diethylamine or diisopropylamine.

7. The process according to claim 1 or 2, wherein said magnesium enolate compound is obtained by reacting a corresponding 2-halogeno cyclohexanone with metallic magnesium.

8. The process according to claim 1 or 2, wherein said magnesium enolate compound is obtained by reacting a corresponding lithium enolate derivative with a magnesium halide.

9. The process according to claim 1 or 2, wherein said magnesium enolate compound is obtained by reacting a corresponding o-trialkylsilyl enol ether derivative with an alkyl magnesium halide.

## Patentansprüche

1. Verfahren zur Herstellung eines Cyclohexylazetidinonderivats der allgemeinen Formel (IV): 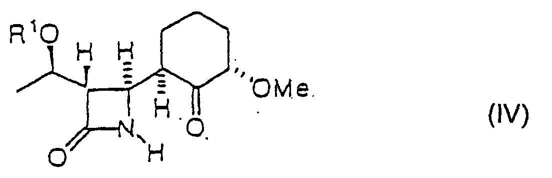 wobei R¹ eine Hydroxyl-Schutzgruppe und Me eine Methylgruppe ist, **dadurch gekennzeichnet, dass** das Verfahren den Schritt umfasst, nämlich Umsetzung eines (3R,4R)-4-Acyloxy-3-[(R)-1-hydroxyethyl]-azetidin-2-on-Derivats der allgemeinen Formel (III): 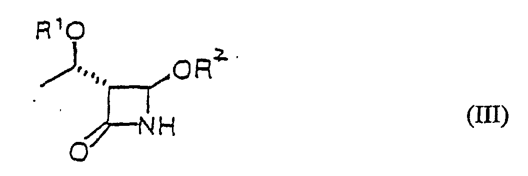 wobei R¹ die vorstehend angegebene Bedeutung hat und R² ein Acylrest ist; mit einer Magnesiumenolatverbindung der allgemeinen Formel (II): 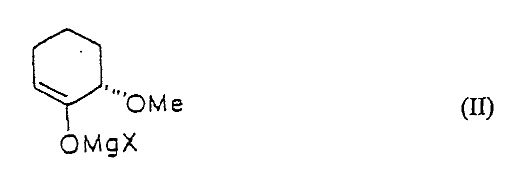 wobei X ein Halogenatom und Me eine Methylgruppe ist, in einem Lösungsmittel.

2. Verfahren gemäß Anspruch 1, wobei das Lösungsmittel entweder Butylacetat oder Toluol ist.

3. Verfahren gemäß Anspruch 1 oder 2, wobei die Magnesiumenolatverbindung durch Umsetzung von (2S)-2-Methoxycyclohexanon der allgemeinen Formel (I): 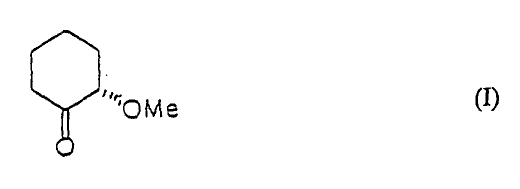 wobei Me eine Methylgruppe ist;
entweder mit einem Produkt, erhalten durch die Umsetzung eines Grignardreagenzes mit einer organischen Base, oder mit einem Grignardreagenz in Gegenwart einer organischen Base erhalten wird.

4. Verfahren gemäß Anspruch 3, wobei das Produkt, das durch die Umsetzung eines Grignardreagenzes mit einer organischen Base erhalten wird, ein Magnesimnamid der allgemeinen Formel (V) ist:
R³R⁴NMgX (V)
wobei die Reste R³ und R⁴ unabhängig voneinander gleich oder verschieden sein können und ein niederer Alkylrest mit 1 bis 6 Kohlenstoffatomen, ein niederer Alkenylrest mit 1 bis 6 Kohlenstoffatomen, ein substituierter oder nicht-substituierter Aralkylrest oder ein substituierter oder nicht-substituierter Phenylrest sind und X ein Halogenatom ist.

5. Verfahren gemäß Anspruch 3, wobei die organische Base ein sekundäres Amin der allgemeinen Formel (VII) ist:
R³R⁴NH (VII)
wobei R³ und R⁴ wie in Anspruch 4 definiert sind, und;
das Grignardreagenz die allgemeine Formel (VIII) aufweist:
R⁵MgX (VIII)
wobei R⁵ die gleiche Bedeutung hat wie R³ oder R⁴ und die Reste R³, R⁴ und R⁵ gleich oder verschieden sein können; und X ein Halogenatom ist.

6. Verfahren gemäß einem der Ansprüche 3 bis 5, wobei die organische Base oder das sekundäre Amin entweder Diethylamin oder Diisopropylamin ist.

7. Verfahren gemäß Anspruch 1 oder 2, wobei die Magnesiumenolatverbindung durch Umsetzung eines entsprechenden 2-Halogencyclohexanons mit metallischem Magnesium erhalten wird.

8. Verfahren gemäß Anspruch 1 oder 2, wobei die Magnesiumenolatverbindung durch Umsetzung eines entsprechenden Lithiumenolatderivats mit einem Magnesiumhalogenid erhalten wird.

9. Verfahren gemäß Anspruch 1 oder 2, wobei die Magnesiumenolatverbindung durch Umsetzung eines entsprechenden o-Trialkylsilylenoletherderivats mit einem Alkylmagnesiumhalogenid erhalten wird.

## Revendications

1. Procédé de préparation d'un dérivé de cyclohexylazétidinone ayant la formule générale (IV) suivante : 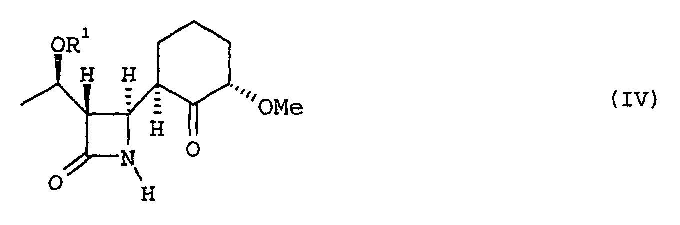 dans laquelle R¹ est un groupe protecteur du groupe hydroxyle et Me est le groupe méthyle,
**caractérisé en ce que** ledit procédé comprend l'étape suivante :
réaction dans un solvant du dérivé de (3R,4R)-4-acyloxy-3-[(R)-1-hydroxyéthyl]-azétidine-2-one ayant la formule générale (III) suivante : 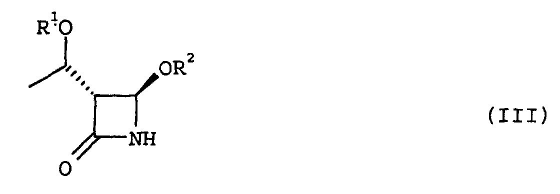 dans laquelle R¹ est le même groupe que celui décrit ci-dessus et R² est un groupe acyle ;
avec un composé énolate de magnésium ayant la formule générale (II) suivante : 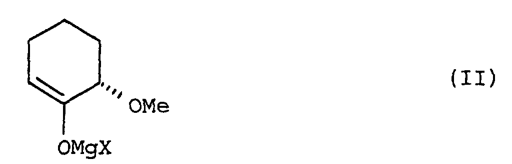 dans laquelle X est un atome d'halogène et Me est le groupe méthyle.

2. Procédé selon la revendication 1, dans lequel ledit solvant est soit l'acétate de butyle soit le toluène.

3. Procédé selon la revendication 1 ou 2, dans lequel ledit composé énolate de magnésium est obtenu par la réaction du (2S)-2-méthoxycyclohexanone ayant la formule générale (I) suivante : 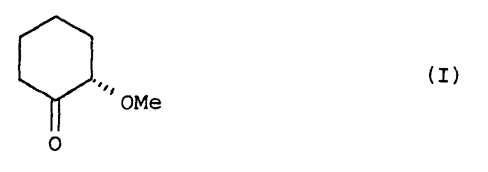 dans laquelle Me est le groupe méthyle ;
soit avec un produit obtenu par la réaction d'un réactif de Grignard avec une base organique, soit avec un réactif de Grignard en présence d'une base organique.

4. Procédé selon la revendication 3, dans lequel ledit produit obtenu par la réaction d'un réactif de Grignard avec une base organique est un amide de magnésium ayant la formule générale (V) suivante :
R³R⁴NMgX (V)
dans laquelle chaque R³ et R⁴ est un groupe alkyle inférieur ayant entre 1 et 6 atomes de carbone, un groupe alcényle inférieur ayant entre 1 et 6 atomes de carbone, un groupe aralkyle substitué ou non substitué, ou un groupement phényle substitué ou non substitué, R³ et R⁴ étant identiques ou différents ;
et X est un atome d'halogène.

5. Procédé selon la revendication 3, dans lequel ladite base organique est une amine secondaire ayant la formule générale (VII) suivante :
R³R⁴NH (VII)
dans laquelle R³ et R⁴ ont la même signification que dans la revendication 4, et;
ledit réactif de Grignard possède la formule générale (VIII) suivante :
R⁵MgX (VIII)
dans laquelle R⁵ a la même signification que R³ ou R⁴, les groupes R³, R⁴ et R⁵ étant identiques ou différents; et X est un atome d'halogène.

6. Procédé selon l'une quelconque des revendications 3 à 5, dans lequel ladite base organique ou amine secondaire est soit la diéthylamine soit la diisopropylamine.

7. Procédé selon la revendication 1 ou 2, dans lequel ledit composé énolate de magnésium est obtenu par la réaction d'un 2-halogéno cyclohexanone correspondant avec du magnésium métallique.

8. Procédé selon la revendication 1 ou 2, dans lequel ledit composé énolate de magnésium est obtenu par la réaction d'un dérivé énolate de lithium correspondant avec un halogénure de magnésium.

9. Procédé selon la revendication 1 ou 2, dans lequel ledit composé énolate de magnésium est obtenu par la réaction d'un dérivé d'éther de o-trialkylsilyle d'énol avec un halogénure de magnésium alkyle.
